# EUROPÄISCHE PATENTSCHRIFT

(11) **EP 1 410 819 B1**
(45) Veröffentlichungstag und Bekanntmachung des Hinweises auf die Patenterteilung: **04.12.2013**
(21) Anmeldenummer: 03021967.9
(22) Anmeldetag: 30.09.2003
(51) Int. Cl.: A61M 5/31, A61M 5/50

(54) **Originalitätsverschluss für eine Spritze**
Warranty seal for a syringe
Fermeture anti-réutilisation pour une seringue

(30) Priorität: 15.10.2002 DE 10247965
(43) Veröffentlichungstag der Anmeldung: 21.04.2004
(73) Patentinhaber: Transcoject GmbH, 24539 Neumünster (DE)
(72) Erfinder: Heinz, Jochen, Dr., 24146 Kiel (DE); Rolle, Alexander, Dr., 24637 Schillsdorf (DE); Schilling, Dieter, 24613 Aukrug-Innien (DE)
(74) Vertreter: Vollmann, Heiko

(56) Entgegenhaltungen:
- EP-A- 0 737 485
- WO-A-96/32441
- DE-A- 19 956 243
- US-A- 5 506 015
- US-A- 5 785 691
- US-A- 5 989 227

## Beschreibung

Die Erfindung betrifft einen Originalitätsverschluss für eine Spritze gemäß den im Oberbegriff des Anspruchs 1 angegebenen Merkmalen.

Bei Spritzen, insbesondere vorgefüllten Spritzen, ist ein Originalitätsverschluss vorgesehen, der für den Anwender die Originalität, d. h. die Unversehrtheit des Verschlusses und damit des in der Spritze abgefüllten Mittels gewährleistet. Derartige Originalitätsverschlüsse zählen zum Stand der Technik und sind beispielsweise aus der EP 0 737 485 oder aus der EP 0 397 951 A1 bekannt. Bei der dort beschriebenen Spritze handelt es sich um eine solche mit Glaszylinder, an deren Ende ein aus Kunststoff bestehendes Bauteil angebracht ist, welches einen Lueranschluss aufweist, auf den später die Kanüle in an sich bekannter Weise aufgesteckt wird oder über den die Spritze in sonstiger Weise angeschlossen wird. Dieser Lueranschluss ist mit einem Stopfen dicht verschlossen, welcher das freie Ende sowohl innen als auch außen vollständig umgreift. Gehalten wird dieser Stopfen von einer Kunststoffkappe, welche über einen Trennsteg mit einem Ring als Fixierbauteil verbunden ist. Dieser Ring weist einen Vorsprung auf, der in einer entsprechenden Ausnehmung am anschlussseitigen Ende der Spritze eingreift und diesen dort festhält. Dabei sind Kappe, Trennsteg und Ring typischerweise einstückig als Spritzgussteil ausgebildet und werden über das anschlussseitige Ende der Spritze geschoben, bis der Vorsprung am Ring am anschlussseitigen Ende einrastet.

Problematisch bei solchen Originalitätsverschlüssen, welche rastend auf das anschlussseitige Ende der Spritze aufgeschoben werden, ist, dass die Fertigungstoleranzen vergleichsweise eng sind um sicherzustellen, dass beim Abnehmen der Kappe tatsächlich der Trennsteg bricht und nicht die Verbindung zwischen dem fixierenden Ring und dem anschlussseitigen Ende der Spritze. Dann nämlich kann der Verschluss ohne weiteres wieder aufgesetzt werden, ohne dass die Originalität und damit die Sterilität des Produktes gewährleistet ist. Darüber hinaus besteht die Gefahr, dass der Trennsteg durch Einknicken bzw. Überdehnen schon beim Aufsetzen bricht. Darüber hinaus ist der Trennsteg häufig so massiv ausgebildet, dass ein Abnehmen der Kappe durch Aufbringen von Zugkräften allein nicht ausreicht, dass vielmehr die Kappe gegenüber dem fixierenden Ring durch Handkraft zusätzlich verdreht werden muss. Dann jedoch sind beide Hände erforderlich, eine zum Fassen des fixierenden Rings und die andere zum Fassen der Kappe. Abgesehen davon, dass dies umständlich ist, besteht insbesondere bei den hier in Rede stehenden medizinischen Spritzen stets die Gefahr, dass hierdurch Keime in diesen Bereich gelangen. Eine dünnere Dimensionierung des Trennstegs ist häufig gar nicht möglich oder werkzeugtechnisch nur schwierig zu realisieren, da nämlich der fixierende Ring in der Regel über den Trennsteg beim Spritzvorgang mit Kunststoff gefüllt werden muss, so dass schon aus diesem Grund ein gewisser Mindestquerschnitt vorhanden sein muss, um ein vollständiges Füllen des fixierenden Rings beim Spritzvorgang sicherzustellen.

Vor diesem Hintergrund üegt der Erfindung die Aufgabe zugrunde, einen Originalitätsverschluss der eingangs genannten Art dahingehend zu verbessern, dass er einerseits kostengünstig herstellbar ist, jedoch andererseits zuverlässig die Unversehrtheit des Verschlusses garantiert, solange sein Trennsteg unversehrt ist.

Diese Aufgabe wird gemäß der Erfindung durch die in Anspruch 1 angegebenen Merkmale gelöst. Vorteilhafte Ausgestaltungen der Erfindung sind in den Unteransprüchen, der nachfolgenden Beschreibung und der Zeichnung angegeben.

Grundgedanke der vorliegenden Erfindung ist es, den Originalitätsverschluss nicht mehr durch Rastverbindung, sondern vielmehr eine stoffschlüssige Verbindung mit dem anschlussseitigen Ende der Spritze zu verbinden. Die stoffschlüssige Verbindung erfolgt durch Schweißen. Dabei ist der Trennsteg in an sich bekannter Weise mit einem Fixierbauteil verbunden, das dann durch Schweißen mit dem anschlussseitigen Ende der Spritze verbunden ist. Durch die gemäß der Erfindung vorgesehene stoffschlüssige Verbindung, die erst nach Aufschieben der Kappe hergestellt wird, kann eine wesentlich haltbarere Verbindung geschaffen werden, als dies bei den bisher bekannten Rastverbindungen möglich ist. Dadurch können die Bauteile mit größerer Toleranz hergestellt werden, was hinsichtlich der Fertigungskosten von Vorteil ist. Der Trennsteg kann den Anforderungen entsprechend dimensioniert werden, so dass die Öffnungskräfte gezielt einstellbar sind. Auch die Werkzeugkosten können hierdurch reduziert werden.

Erfindungsgemäß sind Kappe, Trennsteg und Fixierbauteil einstückig als Spritzgussteil ausgebildet, wobei dann das Fixierbauteil nach Aufsetzen der Kappe durch Schweißen mit dem anschlussseitigen Ende der Spritze fest verbunden wird. Bei entsprechender Auslegung des Fixierbauteils und des anschlussseitigen Endes der Spritze kann der Schweißvorgang in einfacher Weise ausgeführt werden, da keine besonderen Anforderungen hinsichtlich Toleranzen bestehen. Durch eine umlaufende Schweißnaht kann in der Regel ein so stabiler Verbund zwischen Fixierbauteil und anschlussseitigem Ende der Spritze hergestellt werden, dass auch bei ungünstiger Dimensionierung des Trennstegs stets sichergestellt ist, dass die Sollbruchstelle durch den Trennsteg und nicht durch andere Bauteile oder die Schweißnaht gebildet ist.

Ein solcher Ring kann beispielsweise bei einer Spritze mit Luerlockanschluss ohne weiteres an dem spritzenseitigen Zylinderabschnitt angeschweißt werden, der auf seiner Innenseite das Gewinde des Luerlockanschlusses trägt. Unter Zylinderabschnitt im Sinne der Erfindung ist stets ein Hohlzylinder, also ein ringförmiger Körper, zu verstehen.

Besonders vorteilhaft ist es, wenn das ringförmige Fixierbauteil zum freien Ende des Zylinderabschnitts hin verjüngend zulaufend ausgebildet ist, es sich also beispielsweise um einen konusförmigen Ring handelt, da dann das nach Abreißen der Kappe verbleibende Bauteil geeignet ist, das unmittelbare Aufschieben eines Schlauches auf den Luerlockanschluss zu erleichtern und den Schlauch gegenüber dem Außenumfang des Luerlockanschlusses durch diesen erhabenen Ring abzudichten. Der Ring bildet dann somit nicht nur Fixierbauteil, sondern verbessert zugleich die Einsatzmöglichkeiten der Spritze.

Fertigungstechnisch besonders vorteilhaft ist es, wenn das Fixierbauteil durch den Zylinderabschnitt des Luerlockanschlusses selbst gebildet ist. Dann kann nämlich das anschlussseitige Ende der Spritze hinterschneidungsfrei geformt werden, d. h. mit einem vergleichsweise wenig aufwändigen Werkzeug hergestellt werden. Dies ist insbesondere dann von Vorteil, wenn der spritzenseitige Anschluss zusammen mit dem Spritzenzylinder hergestellt wird, wie dies bei Kunststoffspritzen bekannt ist. Die Schweißverbindung zwischen Zylinderabschnitt und dem anschlussseitigen Ende des Spritzenzylinders verbindet dann nicht nur das Fixierbauteil mit dem Spritzenzylinder, sondern dient auch gleichzeitig als konstruktive Verbindung zwischen Zylinderabschnitt des Luerlockanschlusses und dem Spritzenzylinder, erfüllt also insoweit ohnehin konstruktiv erforderliche Maßnahmen. Der Trennsteg ist dann an geeigneter Stelle, beispielsweise stirnseitig des Zylinderabschnitts oder nahe dieser Stirnseite angeformt.

Wie bereits weiter oben ausgeführt ist, ist es von Vorteil, das Fixierbauteil beispielsweise als verjüngend zulaufenden Ring auszugestalten, um auf diese Weise das Aufschieben eines Schlauches sowie die Abdichtung zwischen Schlauch und Zylinderabschnitt zu ermöglichen. Dabei kann entweder dieses ringförmige Fixierbauteil am Zylinderabschnitt des Luerlockanschlusses angeschweißt sein oder aber, wenn der Zylinderabschnitt des Luerlockanschlusses selbst das Fixierbauteil bildet, an diesem zusätzlich angeformt sein.

Um sicherzustellen, dass die Kappe bei Anwendung einer definierten Kraft, in der Regel der Handkraft, sich vom spritzenseitigen Ende löst, d. h. der Trennsteg bricht, ist es zweckmäßig, diesen entsprechend auszubilden. Vorteilhaft ist der Trennsteg umlaufend ausgebildet, da er dann zugleich auch noch einen hermetischen Abschluss zwischen Fixierbauteil und Kappe bildet. Er kann jedoch auch unterbrochen ausgebildet sein, so dass sich eine Vielzahl von Einzelstegen bildet nach Art einer Perforation. Bevorzugt ist er jedoch gleichmäßig verteilt über den Umfang ausgeführt, damit die Kappe von allen Richtungen gleich leicht bzw. schwer entfernt werden kann. Es können auch mehrere Trennstege vorgesehen sein. Um beispielsweise ein Abbrechen an einer gezielten Sollbruchstelle zu ermöglichen, kann der Trennsteg auch nur einseitig vorgesehen sein.

Die Schweißverbindung zwischen dem Fixierbauteil und dem anschlussseitigen Ende der Spritze wird vorzugsweise durch Ultraschweißen gebildet, sie kann jedoch auch durch Vibrationsschweißen, Induktionsschweißen oder andere geeignete Schweißverfahren zum Erzielen eines Stoffschlusses gebildet sein. Da das anschlussseitige Ende der Spritze zusammen mit dem Spritzenzylinder als Kunststoffspritzgussteil einstückig ausgebildet ist, sind für die gesamte Spritze nur wenig Bauteile und entsprechend wenig Montageschritte erforderlich.

Die über den Fixierring angebrachte Kappe kann, wie an sich bekannt, einen elastischen Stopfen integriert haben, der dichtend das anschlussseitige Ende der Spritze abdichtet. Dieser Stopfen kann entweder als gesondertes Bauteil in die Kappe integriert sein oder zusammen mit der Kappe als einstückiges Bauteil extrudiert sein. Gemäß der Erfindung kann es jedoch auch vorgesehen sein, den vergleichsweise hartelastischen Kappenwerkstoff unmittelbar zum Abdichten des Lueranschlusses zu benutzten, indem beim Anschweißen der Kappe diese durch Aufschmelzen des Materials gezielt verkürzt und damit unter Vorspannung gesetzt wird, so dass die Innenseite der Kappe mit dieser Vorspannkraft an der Stirnseite des Lueranschlusses anliegt. Es ist dann ein elastischer Werkstoff zum Abschluss entbehrlich.

Die Spritze, insbesondere der spritzenzylinderseitige Teil wird vorteilhaft aus Polyolefinen hergestellt, bevorzugt Polypropylen (PP), Cykloolefinpolymeren (COP) oder anderen Barrierekunststoffen

Die Erfindung ist nachfolgend anhand eines in der Zeichnung dargestellten Ausführungsbeispiels näher erläutert. Es zeigen:
- Fig. 1: in schematisch vereinfachter Schnittdarstellung einen Längsschnitt durch das anschlussseitige Ende eines Spritzenzylinders mit Luerlockanschluss und Kappe als Originalitätssicherung,
- Fig. 2: eine alternative Ausführungsform in Darstellung nach Fig. 1,
- Fig. 4: eine Ausführungsvariante für eine Spritze mit Lueranschluss in Darstellung nach Fig. 1,
- Fig. 5: eine Ausführungsform, bei der der Lueranschluss mit einem konisch zulaufenden Ring am Außenumfang versehen ist, in Darstellung nach Fig. 1,
- Fig. 6: die Ausführung nach Fig. 5 in alternativer Bauweise und in Darstellung nach Fig. 1 und
- Fig. 7: eine Einzelheit der Fig. 6 in vergrößerter Darstellung.

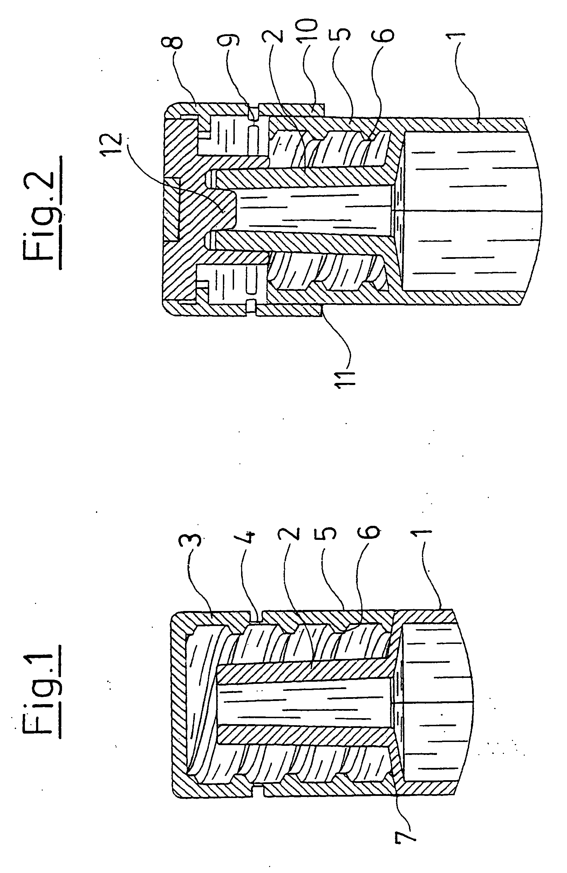

In Fig. 1 ist das anschlussseitige Ende eines Spritzenzylinders 1 dargestellt, der in einen Lueranschluss 2 mündet, der in an sich bekannter Weise zum Aufsetzen einer Kanüle vorgesehen ist. Der Lueranschluss 2 und der Spritzenzylinder 1 sind einstückig als Kunststoffspritzgussteil ausgebildet.

Als Originalitätsverschluss ist eine den Lueranschluss 2 am freien Ende mit Abstand überdeckende Kappe 3 vorgesehen, welche über einen umlaufenden Trennsteg 4 mit einem Zylinderabschnitt 5 verbunden ist, der an seiner Innenseite ein Gewinde 6 aufweist und der den Lueranschluss 2 umgebend an das anschlussseitige Ende des Spritzenzylinders 1 anschließt. Kappe 3, Trennsteg 4 und Zylinderabschnitt 5 sind ebenfalls einstückig als Kunststoffspritzgussteil ausgebildet. Der Zylinderabschnitt 5 ist an seiner zum Spritzenzylinder 1 weisenden Stirnseite mit diesem durch Ultraschallschweißen stoffschlüssig und fest verbunden. Die Schweißnaht ist mit 7 gekennzeichnet.

Der so gebildete Originalitätsverschluss ist für eine nicht vorgefüllte oder mit pastösem Stoff vorgefüllte Spritze vorgesehen. Zum Öffnen des Verschlusses wird die Kappe 3 mittels Handkraft vom Zylinderabschnitt 5 entfernt, wobei der Trennsteg 4, der eine Sollbruchstelle darstellt, zerstört wird. Die Anordnung und Ausbildung des Zylinderabschnitts 5 in Bezug auf den Lueranschluss 2 sind so gewählt, dass nach Entfernen der Kappe 3 ein Luerlockanschluss gebildet ist.

Bei der anhand von Fig. 2 dargestellten Ausführungsvariante ist am anschlussseitigen Ende des Spritzenzylinders 1 ein vollständiger Luerlockanschluss bestehend aus dem inneren Lueranschluss 2 und einem Zylinderabschnitt 5 mit Innengewinde 6 angeformt. Bei dieser Ausführungsvariante ist eine Kappe 8 vorgesehen, welche über einen abschnittsweise unterbrochenen Trennsteg 9 mit einem Fixierbauteil 10 in Form eines Rings verbunden ist. Der Ring 10 übergreift den Endabschnitt des Zylinderabschnitts 5 des Luerlockanschlusses am Außenumfang. Die Bauteile sind im überlappenden Bereich durch Ultraschallschweißen fest miteinander verbunden. Die Schweißnaht ist mit 11 gekennzeichnet.

Innerhalb der Kappe 8 ist ein Stopfen 12 eingegliedert, welcher den Lueranschluss sowohl innen als auch außen endseitig umgreift und dicht verschließt. Der Stopfen 12 ist aus einem weich-elastischen Material gebildet, wohingegen die Kappe 8, der Trennsteg 9 und der Ring 10 aus einem härteren Kunststoff ebenso wie der Spritzenzylinder 1 mit dem daran angeformten Luerlockanschluss hergestellt sind.

Die Ausbildung nach Fig. 2 ist insbesondere für eine vorgefüllte Spritze geeignet. Zum Öffnen wird die Kappe 8 vom anschlussseitigen Spritzenende abgezogen, wodurch der Trennsteg 9 bzw. die Vielzahl über den Umfang verteilt angeordneten Trennstege abreißen und zusammen mit dem Stopfen 12 entfernt werden können.

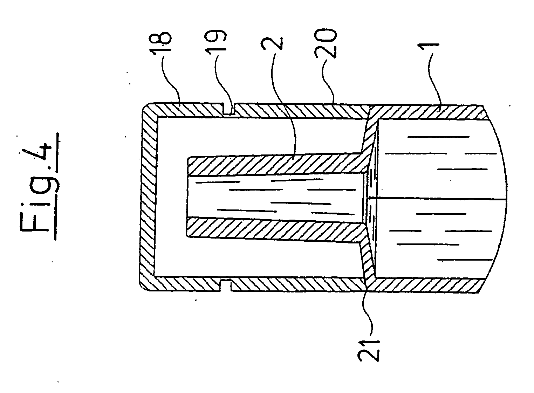

Die Ausführung gemäß Fig. 4 zeigt einen Originalitätsverschluss für eine nicht vorgefüllte Spritze mit einem Spritzenzylinder 1 mit Lueranschluss 2. Eine becherförmige Kappe 18 ist über einen umlaufenden Trennsteg 19 mit einem Fixierbauteil 20 in Form eines Zylinderabschnitts einstückig und als Kunststoffspritzgussteil ausgebildet. Das Fixierbauteil 20 ist in ähnlicher Weise wie bei der Ausführung nach Fig. 1 stirnseitig mit dem Spritzenzylinder 1 durch Schweißen verbunden. Die Schweißnaht ist mit 21 gekennzeichnet. Die Kappe 18 wird durch Handkraft vom Fixierbauteil 20 getrennt, wobei der Trennsteg 19 zerstört wird und das Ende des Lueranschlusses 2 zugänglich wird.

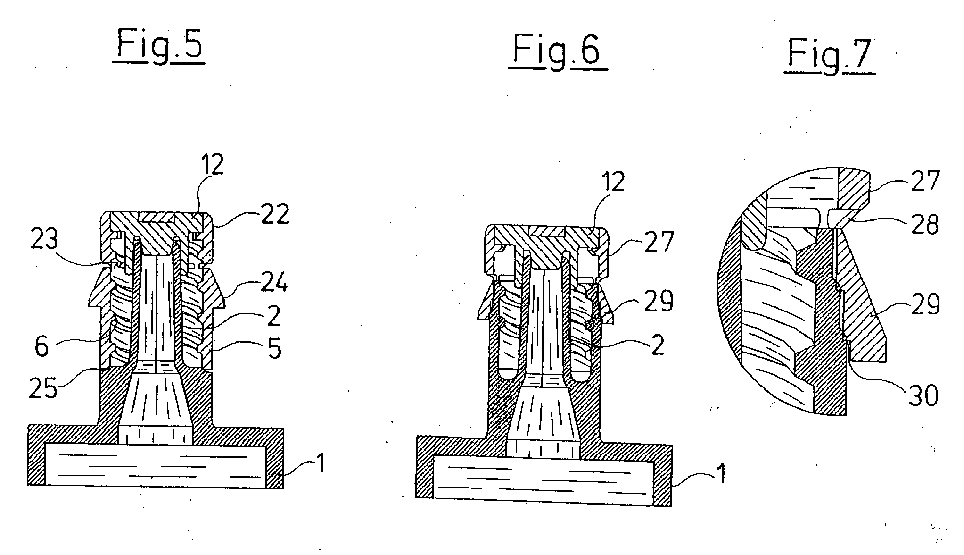

Das anhand von Fig. 5 dargestellte anschlussseitige Ende eines Spritzenzylinders 1 mündet in einen Lueranschluss 2 und ist einstückig mit diesem ausgebildet. Eine Kappe 22, ähnlich der anhand von Fig. 2 beschriebenen, ist über einen unterbrochenen Trennsteg 23 mit einem Zylinderabschnitt 5 mit Innengewinde 6 verbunden, der Teil des nach Abnehmen der Kappe 22 entstehenden Luerlockanschlusses ist. Am Auβenumfang des Zylinderabschnitts 5 ist nahe seinem freien Ende ein im Querschnitt verjüngend zulaufender Ring 24 angeformt, der zusammen mit dem Zylinderabschnitt 5, dem Trennsteg 23 und der Kappe 22 als Spritzgussteil ausgebildet ist. Auch bei dieser Ausführungsform weist die Kappe 22 einen Stopfen 12 auf, der zum dichten Abschluss des Lueranschlusses 2 dient. Die Verbindung des Zylinderabschnitts 5 mit dem anschlussseitigen Ende des Spritzenzylinders 1 erfolgt über eine stirnseitige Schweißnaht 25, ähnlich der anhand von Fig. 1 beschriebenen Ausführung.

Auch hier wird durch Handkraft die Kappe 22 mit dem darin eingegliederten Stopfen entfernt, nachdem der Trennsteg 23 als Sollbruchstelle vom Zylinderabschnitt 5 abgerissen ist. Dann ergibt sich spritzenanschlussseitig ein Luerlockanschluss. Der konisch zulaufende Ring 24 dient dazu, dass auf den Außenumfang des Luerlockanschlusses unmittelbar ein Schlauch aufgeschoben werden kann. Dieser Ring erleichtert das Aufschieben, die im hinteren Bereich gebildete Kante, die auch als Ringabschnitt oder Rundung ausgebildet sein kann, bewirkt eine erhöhte Flächenpressung zwischen der Innenseite des Schlauches und der Außenseite des Ringes, wodurch Dichtwirkung erzielt wird.

Die anhand der Figuren 6 und 7 dargestellte Ausführungsvariante unterscheidet sich von der vorbeschriebenen dadurch, dass in diesem Fall das anschlussseitige Ende des Spritzenzylinders 1 einstückig mit einem Luerlockanschluss ausgebildet ist. Die Kappe 27 ist über einen Trennsteg 28, der sich über den gesamten stirnseitigen Umfang erstreckt, der jedoch abschnittsweise unterbrochen ist, mit einem Fixierbauteil 29 verbunden und einstückig als Spritzgussteil ausgebildet, das, wie sich aus Fig. 7 ergibt, im Wesentlichen die Form des konischen Rings 24 aufweist. Bei dieser Ausführung ist das Fixierbauteil durch Schweißen am Außenumfang des Zylinderabschnitts 5 befestigt, die Schweißnaht ist mit 30 gekennzeichnet. Das Fixierbauteil 29 hat, nachdem die Kappe 27 durch Aufreißen des Trennstegs 28 entfernt worden ist, die gleiche Funktion wie der anhand von Fig. 5 beschriebene konische Ring 24.

Wie die vorstehenden Ausführungsbeispiele verdeutlichen, kann die vorliegende Erfindung neben einem funktionssicheren Originalitätsverschluss auch noch weitere Funktionen beinhalten, die insbesondere in Kombination fertigungstechnisch günstig herstellbar sind. Es versteht sich, dass die vorbeschriebenen Einzelmerkmale nur beispielhaft zu verstehen sind und daher auch, soweit sinnvoll, praktisch beliebig miteinander kombinierbar sind. So können beispielsweise auch die ohne Stopfen dargestellten Ausführungsvarianten mit einem solchen hergestellt werden oder umgekehrt. Allen gemeinsam ist, dass aufgrund der stoffschlüssigen festen Schweißverbindung zuverlässig sichergestellt ist, dass beim Abtrennen einer Kappe stets der Trennsteg und nicht das Fixierbauteil (wie beim Stand der Technik) gelöst wird. Auf diese Weise ist nach Entfernen der Kappe der Originalitätsverschluss zuverlässig zerstört.

### Bezugszeichenliste

- 1: - Spritzenzylinder
- 2: - Lueranschluss
- 3: - Kappe (Fig. 1)
- 4: - Trennsteg
- 5: - Zylinderabschnitt
- 6: - Gewinde
- 7: - Schweißnaht
- 8: - Kappe (Fig. 2)
- 9: - unterbrochener Trennsteg
- 10: - Fixierbauteil, Ring
- 11: - Schweißnaht
- 12: - Stopfen
- 18: - Kappe (Fig. 4)
- 19: - Trennsteg
- 20: - Fixierbauteil
- 21: - Schweißnaht
- 22: - Kappe (Fig. 5)
- 23: - Trennsteg
- 24: - Ring
- 25: - Schweißnaht
- 27: - Kappe (Fig. 6)
- 28: - Trennsteg
- 29: - Fixierbauteil
- 30: - Schweißnaht

## Patentansprüche

1. Originalitätsverschluss für eine Spritze mit Luer- oder Luerlockanschluss, bei der mindestens das anschlussseitige Ende aus Kunststoff besteht, mit einer mindestens den Lueranschluss (2) übergreifenden Kappe (3), die über mindestens einen Trennsteg (4) mit der Spritze (1) verbunden ist, wobei die Kappe (3) als Kunststoffspritzgussteil ausgebildet ist und der Trennsteg (4) eine Sollbruchstelle bildet, die beim Entfernen der Kappe (3) von der Spritze (1) bricht, **dadurch gekennzeichnet, dass** die Kappe (3) über den Trennsteg (4) mit einem Fixierbauteil verbunden ist, welches durch Schweiβen (7) mit dem anschlussseitigen Ende der Spritze (1) verbunden ist und dass Kappe (3), Trennsteg (4) und Fixierbauteil (5) einstückig als Spritzgussteil ausgebildet sind.

2. Originalitätsverschluss nach Anspruch 1, **dadurch gekennzeichnet, dass** das Fixierbauteil durch einen Ring (29) gebildet ist, welcher an einem spritzenseitigen Zylinderabschnitt (5) mit Innengewinde (6) angeschweißt ist, der den Lueranschluss (2) umgibt und Teil des Luerlockanschlusses bildet.

3. Originalitätsverschluss nach einem der vorgehenden Ansprüche, **dadurch gekennzeichnet, dass** das ringförmige Fixierbauteil (29) einen zum freien Ende des Zylinderabschnitts (5) verjüngend zulaufenden Querschnitt aufweist.

4. Originalitätsverschluss nach einem der vorhergehenden Ansprüche, **dadurch gekennzeichnet, dass** der Zylinderabschnitt (5) des Luerlockanschlusses das Fixierbauteil bildet.

5. Originalitätsverschluss nach einem der vorhergehenden Ansprüche **dadurch gekennzeichnet, dass** der das Fixierbauteil bildende Zylinderabschnitt (5) an seinem Außenumfang einen im Querschnitt zum freien Ende des Lueranschlusses (2) verjüngend zulaufenden Ring (24) aufweist.

6. Originalitätsverschluss nach einem der vorhergehenden Ansprüche, **dadurch gekennzeichnet, dass** der Trennsteg (9) Unterbrechungen aufweist, die gleichmäßig über den Umfang verteilt angeordnet sind.

7. Originalitätsverschluss nach einem der vorhergehenden Ansprüche, **dadurch gekennzeichnet, dass** die Kappe (22) aus zwei unterschiedlichen Kunststoffen aufgebaut ist und einen weichelastischen Stopfen (12) aufweist.

8. Originalitätsverschluss nach einem der vorhergehenden Ansprüche, **dadurch gekennzeichnet, dass** die Kappe (22) und der weichelastische Stopfen (12) als einstückiges Kunststoffspritzgussteil ausgebildet sind.

9. Originalitätsverschluss nach einem der vorhergehenden Ansprüche, **dadurch gekennzeichnet, dass** die Kappe mit definierter Vorspannung angeschweißt ist, derart, dass diese den Lueranschluss dichtend abschließt.

10. Originalitätsverschluss nach einem der vorhergehenden Ansprüche, **dadurch gekennzeichnet, dass** die durch Schweißen miteinander zu verbindenden Bauteile aus Polyolefinen, bevorzugt aus Polypropylen (PP) oder Cykloolefinpolymeren (COP) bestehen.

## Claims

1. A tamper-evident closure for a syringe with a Luer connection or a Luer lock connection, with which at least the connection-side end consists of plastic, with a cap (3) which engages over at least the Luer connection (2) and which is connected to the syringe (1) via at least one separating web (4), wherein the cap (3) is designed as a plastic injection moulded part, and the separating web (4) forms a break location which breaks on removing the cap (3) from the syringe (1), **characterised in that** the cap (3) is connected via the separating web (4) to a fixation component which is connected by welding (7) to the connection-side end of the syringe (1), and that the cap (3), the separating web (4) and the fixation component (5) are designed as one piece as an injection moulded part.

2. A tamper-evident closure according to claim 1, **characterised in that** the fixation component is formed by a ring (29) which is welded on a syringe-side cylinder section (5) with an inner thread (6) which surrounds the Luer connection (2) and forms part of the Luer lock connection.

3. A tamper-evident closure according to one of the preceding claims, **characterised in that** the annular fixation component (29) has a cross section tapering to the free end of the cylinder section (5).

4. A tamper-evident closure according to one of the preceding claims, **characterised in that** the cylinder section (5) of the Luer lock connection forms the fixation component.

5. A tamper-evident closure according to one of the preceding claims, **characterised in that** the cylinder section (5) forming the fixation component, on its outer periphery comprises a ring (24) tapering in cross section to the free end of the Luer connection (2).

6. A tamper-evident closure according to one of the preceding claims, **characterised in that** the separating web (9) comprises interruptions which are uniformly arranged over the periphery.

7. A tamper-evident closure according to one of the preceding claims, **characterised in that** the cap (22) is constructed of two different plastics and comprises a soft-elastic plug (12).

8. A tamper-evident closure according to one of the preceding claims, **characterised in that** the cap (22) and the soft-elastic plug (12) are designed as a single-piece plastic injection moulded part.

9. A tamper-evident closure according to one of the preceding claims, **characterised in that** the cap is welded with a defined prestress in a manner such that this sealingly closes the Luer connection.

10. A tamper-evident closure according to one of the preceding claims, **characterised in that** the components to be connected to one another by way of welding consist of polyolefins, preferably polypropylene (PP) or cyclo-olefin polymers (COP).

## Revendications

1. Fermeture inviolable pour seringue comportant un embout Luer ou Luer-Lock, dans laquelle au moins l'extrémité côté embout est constituée d'un matériau plastique, comportant un capuchon (3) recouvrant au moins l'embout Luer (2), capuchon qui est relié à la seringue (1) par l'intermédiaire d'au moins une nervure de séparation (4), le capuchon (3) étant formé sous la forme d'une pièce plastique moulée par injection, et la nervure de séparation (4) formant une zone de rupture qui se rompt quand le capuchon (3) est enlevé de la seringue (1), **caractérisée en ce que** le capuchon (3) est, par l'intermédiaire de la nervure de séparation (4), relié à un élément de fixation, lequel est relié par soudage (7) à l'extrémité côté embout de la seringue (1), et **en ce que** le capuchon (3), la nervure de séparation (4) et l'élément de fixation (5) sont formés sous la forme d'une pièce monobloc moulée par injection.

2. Fermeture inviolable selon la revendication 1, **caractérisée en ce que** l'élément de fixation est formé d'une bague (29), qui est soudée à une partie cylindrique (5), côté seringue, comportant un filetage intérieur (6), qui entoure l'embout Luer (2) et forme une partie de l'embout Luer-Lock.

3. Fermeture inviolable selon l'une des revendications précédentes, **caractérisée en ce que** l'élément de fixation annulaire (29) présente une section transversale qui va se rétrécissant vers l'extrémité libre de la partie cylindrique (5).

4. Fermeture inviolable selon l'une des revendications précédentes, **caractérisée en ce que** la partie cylindrique (5) de l'embout Luer-Lock forme l'élément de fixation.

5. Fermeture inviolable selon l'une des revendications précédentes, **caractérisée en ce que** la partie cylindrique (5) formant l'élément de fixation présente sur sa périphérie extérieure une bague (24), dont la section transversale va se rétrécissant vers l'extrémité libre de l'embout Luer (2).

6. Fermeture inviolable selon l'une des revendications précédentes, **caractérisée en ce que** la nervure de séparation (9) comprend des interruptions qui sont disposées en étant uniformément réparties sur sa périphérie.

7. Fermeture inviolable selon l'une des revendications précédentes, **caractérisée en ce que** le capuchon (22) est constitué de deux matériaux plastiques différents, et comprend un bouchon (12) élastique souple.

8. Fermeture inviolable selon l'une des revendications précédentes, **caractérisée en ce que** le capuchon (22) et le bouchon élastique souple (12) sont formés sous la forme d'une pièce plastique monobloc moulée par injection.

9. Fermeture inviolable selon l'une des revendications précédentes, **caractérisée en ce que** le capuchon est soudé avec une pré-tension définie, de telle sorte que cette dernière obture l'embout Luer d'une manière étanche.

10. Fermeture inviolable selon l'une des revendications précédentes, **caractérisée en ce que** les éléments devant être assemblés l'un à l'autre par soudage sont constitués de polyoléfines, de préférence de polypropylène (PP) ou de polymères de cyclooléfine (COP).
